# EUROPEAN PATENT APPLICATION

(11) **EP 1 516 630 A2**
(43) Date of publication of application: **23.03.2005**
(21) Application number: 04020503.1
(22) Date of filing: 30.10.1998
(51) Int. Cl.: A61K 39/395, A61K 38/13

(54) **Use of anti-il-12 antibodies in transplantation rejection**

(30) Priority: 31.10.1997 US 63853 P
(62) Divisional of application: 98956450.5
(71) Applicant: Wyeth, Madison, New Jersey 07940 (US); Beth Israel Deaconess Medical Center, Boston, MA 02215 (US)
(72) Inventor: Strom, Terry, Brookline, MA 02445 (US); Li, Xiang Chang, Newton, MA 02464 (US); Zand, Martin S., Rochester, NY 14618 (US); Zheng, Xin Xiao, Wellesley, MA 02481 (US)
(74) Representative: VOSSIUS & PARTNER

(57) **Abstract**

Methods are disclosed for reducing or ameliorating chronic rejection of a transplanted tissue by administration of an anti-IL-12 antibody, alone or in combination with other beneficial therapeutic agents.

## Description

### Background of the Invention

Antigen-activated CD4+ Th cells can differentiate into at least two distinct phenotypes. Th1 cells produce IL-2, IFN-, and TNF- and often orchestrate the T cell-dependent cytopathic effects noted in certain autoimmune states, allograft rejection, and delayed-type hypersensitivity responses. In contrast, Th2 cells secrete IL-4, IL-5, IL-6, and IL-10 and are the classic Th cells for the provision of B cell help in Ab production (1). Central to this polarized nature of CD4+ T cells is the role of certain cytokines that act as cross-regulators in Th1 and Th2 differentiation. IL-12 that is produced primarily by activated macrophages/monocytes is a powerful and probably an obligatory cytokine in driving Th1 differentiation (2). Indeed, mice that are deficient for IL-12 (3), the IL-12R1 (4), or STAT-4 (5), a key IL-12 signaling component, manifest impaired Th1 responses. The differentiation of Th2 cells, however, requires IL-4, which is produced by T cells and CD4+NK1.1+ cells (1). As the Th1 and Th2 paradigm holds that the ultimate fate of an immune response is determined as a consequence of whether the Th1 or Th2 is in control of the other, it has been proposed that a Th1 to Th2 immune deviation may be beneficial in certain Th1-mediated pathologic processes. Indeed, a Th1 to Th2 deviation that is produced by treatment with Th2 cytokines or anti-IL-12 mAb is highly beneficial in several models of T cell-dependent autoimmunity (6-12), suggesting an unequivocal role for Th2 cells as an important immune regulatory population in this situation.

Allograft rejection in unmodified recipients is consistently, albeit not universally, associated with a Th1 pattern of immune activation. Moreover, allograft recipients that are treated with tolerizing immunosuppressive regimens often manifest a Th2-type response during the treatment period (13-17). Nonetheless, IL-2-deficient hosts reject allografts despite a strong expression of IL-4 (18), and IL-4-deficient allograft hosts receiving potent immunosuppression can be permanently engrafted (19, 20). Furthermore, attempts to induce permanent engraftment or create a state of allograft tolerance through the administration of long-acting Th2 cytokines (i.e., IL-4Ig and IL-10Ig fusion proteins) or immune deviation via the application of IL-12 antagonists have failed (21, 22). It is puzzling as to why treating allograft recipients with such strategies, which are extremely effective in dampening autoimmunity (6-12), has not proven efficacious in transplantation. Adding to the mystery, the states of neonatal tolerance and "infectious tolerance" to alloantigens can be broken through the administration of anti-IL-4 (23-25).

We have reasoned that T cell-dependent alloimmune and autoimmune responses differ in at least two different and interrelated ways. First, the host response to MHC-mismatched allografts includes responses to directly presented foreign (donor) MHC Ags (i.e., direct Ag presentation), while autoimmunity is characterized by the indirect presentation of autoantigens upon self MHC molecules. Second, the rejection of MHC-incompatible allografts activates more T cell clones than do typical autoimmune responses. It would, therefore, be desirable to determine whether these factors lie at the heart of the paradoxical ability of immune deviation to curtail T cell-dependent autoimmunity while failing to blunt T cell-dependent responses to MHC mismatched-allografts. It would also be desirable to determine whether antagonism of IL-12, preferably by an anti-IL-12 antibody, will inhibit rejection in tissue transplantation.

### Summary of the Invention

The present invention provides a method of reducing or ameliorating chronic rejection of a transplanted tissue in a mammalian subject, comprising administering to said subject a therapeutically effective amount of an anti-IL12 antibody. Preferably, such subject is human. In other preferred embodiments, the transplanted tissue has a mismatch of minor alloantigens with said-subject.

In certain preferred embodiments, the antibody is a polyclonal antibody, a monoclonal antibody, a chimeric antibody, a single-chain antibody, a CDR-grafted antibody, a humanized antibody or fragments thereof which bind IL-12. Preferably, such antibody is a human antibody.

Further embodiments provide for administration of the antibody in combination with at least one other therapeutic agent. Preferably, such other agent is one that reduces or eliminates a secondary co-stimulatory immune signal, such as, for example, a soluble form of CTLA4, an antibody directed to B7.1, an antibody directed to B7.2 or antibody directed to CD28. In other preferred embodiments, the other therapeutic agent is an immunosuppressant, such as, for example, cyclosporin or rapamycin.

### Brief Description of the Drawings

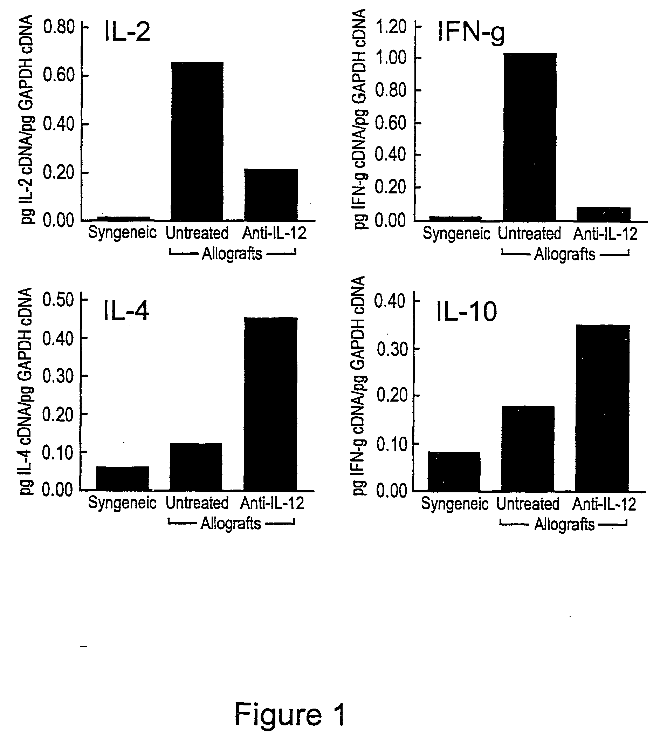

Figure 1. Intragraft expression of Th1 (IL-2 and IFN-) and Th2 (IL-4 and IL-10) gene transcripts in MHC-mismatched islet allografts. The islet allografts were harvested on day 8 posttransplant from untreated or anti-IL-12-treated recipient mice, and the intragraft expression of the cytokine gene transcripts was determined by QRT-PCR. THe results are expressed as picograms of target gene cDNA per picogram of GAPDH cDNA. The representative data that were detected in one of three animals in each group are shown.

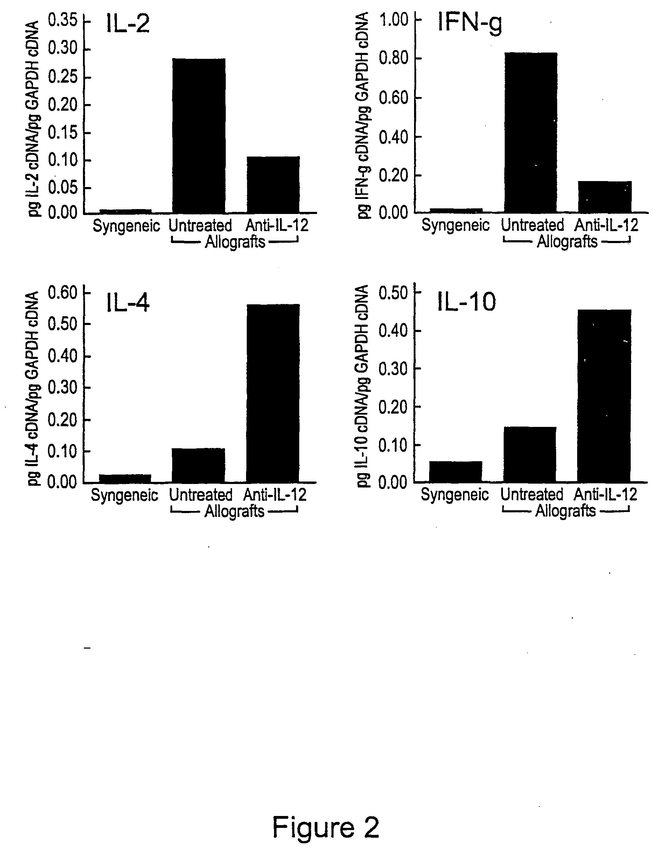

Figure 2. Intragraft expression of Th1 (IL-2 and IFN-) and Th2 (IL-4 and IL-10) gene transcripts in MHC-matched but multiple minor Ag-mismatched islet allografts. The islet allografts were harvested on day 16 posttransplant from untreated or anti-IL-12-treated recipient mice, and the intragraft expression of the cytokine gene transcripts was determined by QRT-PCR. The results are expressed as picograms of target gene cDNA per picogram of GAPDH cDNA. The representative data that were detected in one of three animals in each group are shown.

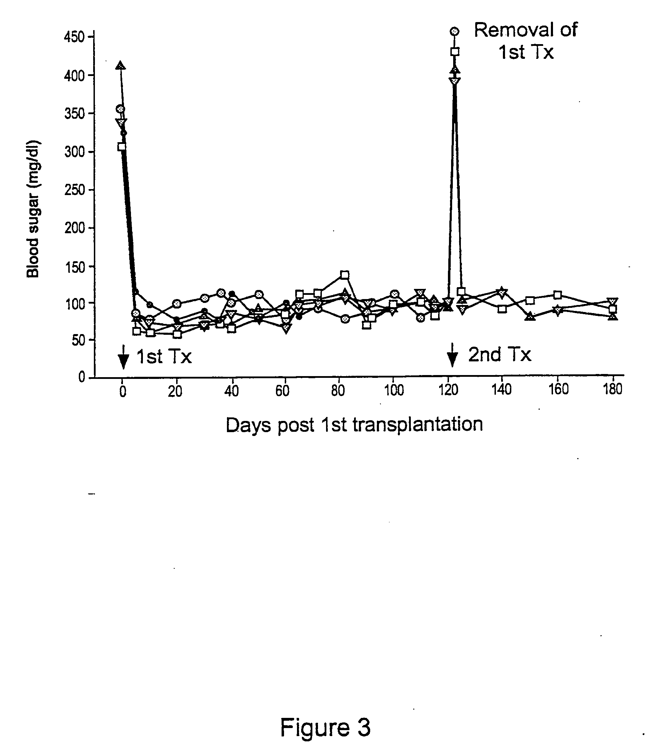

Figure 3. Induction of tolerance in anti-IL-12-treated multiple minor Ag-mismatched recipients. Nephrectomy of the islet graft-bearing kidney on five BALB/c recipients at 120 days posttransplantation led to a sharp rise in blood glucose levels. Nephrectomized BALB/c mice accepted a second DBA/2J islet allograft without any immunosuppression:

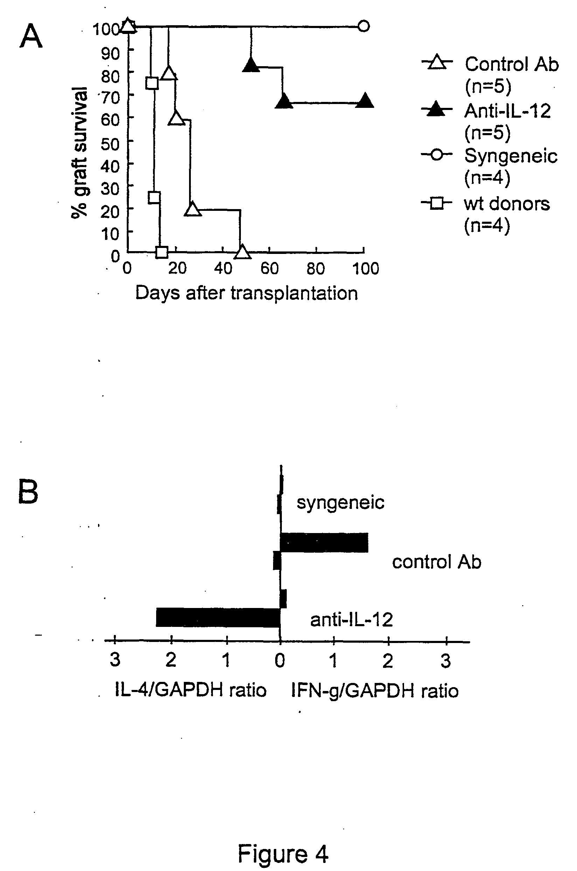

Figure 4. A, Survival of islet allografts from MHC class II KO C57BL/6 mice in thymectomized and CD8+ T cell-depleted BALB/c recipients that were treated with anti-IL-12 mAb. B, Intragraft Th1 (IFN-)/Th2 (IL-4) gene transcript analysis in control Ab- or anti-IL-12-treated recipients.

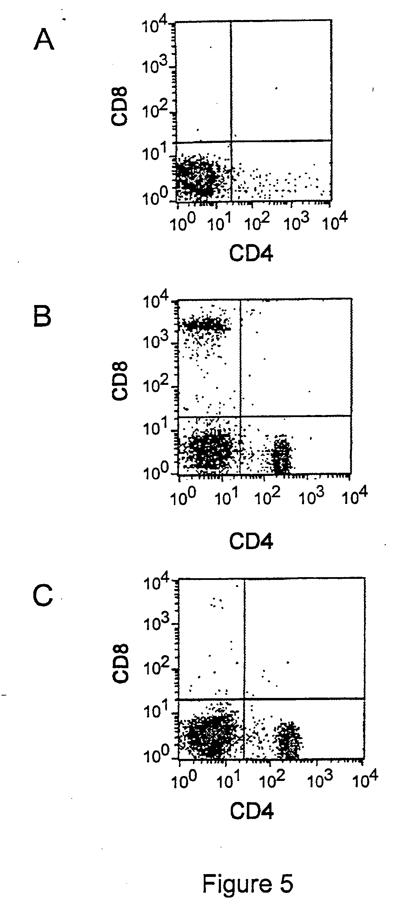

Figure 5. FACS analysis of CD4+ and CD8+ T cells in the peripheral blood of control mice or thymectomized and CD8-depleted recipient mice at the time of allograft rejection. A, isotype control Ab staining; B, unmodified control BALB/c recipients; C, thymectomized and CD8-depleted BALB/c recipients.

### Detailed Description of Preferred Embodiments

As used herein, "anti-IL-12 antibody" includes, without limitation, a polyclonal antibody, a monoclonal antibody, a chimeric antibody, a single-chain antibody, a CDR-grafted antibody, a humanized antibody or fragments thereof which bind IL-12. Such term also includes any other species derived from an antibody or antibody sequence which is capable of binding to IL-12. Preferably, such anti-IL-12 antibody is human.

Anti-IL-12 antibodies can be produced by methods well known to those skilled in the art. For example, monoclonal IL-12 antibodies can be produced by generation of antibody-producing hybridomas in accordance with known methods (see for example, Coding. 1983. Monoclonal antibodies: principles and paractice. Academic Press Inc., New York; Yokoyama. 1992. "Production of Monoclonal Antibodies" in Current Protocols in Immunology. Unit 2.5. Greene Publishing Assoc. and John Wiley & Sons). Polyclonal sera and antibodies to IL-12 can be produced by inoculation of a mammalian subject with IL-12 or fragments thereof in accordance with known methods. Fragments of antibodies, receptors or other reactive peptides can be produced from the corresponding antibodies by cleavage of and collection of the desired fragments in accordance with known methods (see for example, Goding, supra; Andrew et al. 1992. "Fragmentation of Immunoglobulins" in Current Protocols in Immunology. Unit 2.8. Greene Publishing Assoc. and John Wiley & Sons). Chimeric antibodies and single chain antidbodies can also be produced in accordance with known recombinant methods (see for example, 5,169,939, 5,194,594 and 5,576,184). Humanized antibodies can also be made from corresponding murine antibodies in accordance with well known methods (see for example, U.S. Patent Nos. 5,530,101,5,585,089 and 5,693,762).

The anti-IL-12 antibody can also be adminstered in combination with other therapeutic agents which provide benefits in tissue transplantation, such as agents which provide immunosuppression or otherwise downregulate the usual immunresponse attendant to mismatched transplants (such as antagonists of secondary immune co-stimulatory signals). A wide variety of such agents are already known to those skilled in the art and are commercially available, such as, for example, cyclosporin, rapamycin and related compounds. Examples of antagonists of secondary immune co-stimulatory signals include, without limitation, soluble forms of CTLA4, antibodies directed to B7.1, antibodies directed to B7.2 and antibodies directed to CD28.

Pharmaceutical compositions containing an anti-IL-12-antibody which are useful in practicing the methods of the present invention may also contain pharmaceutically acceptable carriers, diluents, fillers, salts, buffers, stabilizers and/or other materials well-known in the art. The term "pharmaceutically acceptable" means a material that does not interfere with the effectiveness of the biological activity of the active ingredient(s) and that is not toxic to the host to which it is administered. The characteristics of the carrier or other material will depend on the route of administration.

It is currently contemplated that the various pharmaceutical compositions should contain about 0.1 micrograms to about 1 milligram per milliliter of the anti-IL-12-antibody.

Administration can be carried out in a variety of conventional ways. Intraperitoneal injection is the preferred method of administration of the anti-IL-12-antibody. Intravenous, cutaneous or sub-cutaneous injection may also be employed. For injection, anti-IL-12-antibody will preferably be administered in the form of pyrogen-free, parenterally acceptable aqueous solutions. The preparation of such parenterally acceptable protein solutions, having due regard to pH, isotonicity, stability and the like, is within the skill of the art.

The amount of anti-IL-12-antibody used for treatment will depend upon the severity of the condition, the route of administration, the reactivity of the anitbody with IL-12 or the activity of the antibody, and ultimately will be decided by the treatment provider. In practicing the methods of treatment of this invention, a therapeutically effective amount of an anti-IL-12-antibody is administered. The term "therapeutically effective amount" means the total amount of each active component of the method or composition that is sufficient to show a meaningful patient benefit (e.g., curing, ameliorating, delaying or preventing onset of, preventing recurrence or relapse of). One common technique to determine a therapeutically effective amount for a given patient is to administer escalating doses periodically until a meaningful patient benefit is observed by the treatment provider. When applied to an individual active ingredient, administered alone, the term refers to that ingredient alone. When applied to a combination, the term refers to combined amounts of the active ingredients that result in the therapeutic effect, whether administered in combination, serially or simultaneously. A therapeutically effective dose of an anti-IL-12 antibody in this invention is contemplated to be in the range of about 0.05 mg/kg to about 25 mg/kg, preferably about 1 mg/kg to about 20 mg/kg, more preferably about 2 mg/kg to about 10 mg/kg. The number of administrations may vary, depending on the individual patient and the severity of the autoimmune condition.

Additional therapeutic agents administered with the anti-IL-12 antibody are administered in accordance with known methods for their administration, such as, for example, instructions disributed with commercially-available products. Such additional therapeutic agents can be administered at the same time as, before or after the time of administration of the anti-IL-12 antibody. Such additional agents can also be adminsitered by the same or different route of administration as the anti-IL-12 antibody.

The following items also characterize the invention:
- Item 1:: A method of reducing or ameliorating chronic rejection of a transplanted tissue in a mammalian subject, comprising administering to said subject a therapeutically effective amount of an anti-IL12 antibody.
- Item 2:: The method as described in item 1 wherein said subject is human.
- Item 3:: The method as described in item 1 wherein said tissue has a mismatch of minor alloantigens with said subject.
- Item 4:: The method as described in item 1 wherein said antibody is administered in combination with at least one other therapeutic agent.
- Item 5:: The method as described in item 4 wherein said other therapeutic agent reduces or eliminates a secondary co-stimulatory immune signal.
- Item 6:: The method as described in item 5 wherein said therapeutic agent is selected from the group consisting of a soluble form of CTLA4, an antibody directed to B7.1, an antibody directed to B7.2 and an antibody directed to CD28.
- Item 7:: The method as described in item 1 wherein said antibody is selected from the group consisting of a polyclonal antibody, a monoclonal antibody, a chimeric antibody, a single-chain antibody, a CDR-grafted antibody, a humanized antibody and fragments thereof which bind IL-12.
- Item 8:: The method as described in item 1 wherein said antibody is a human antibody.
- Item 9:: The method as described in item 4 wherein said other therapeutic agent is an immunosuppressant.
- Item 10:: The method as described in item 9 wherein said immunosuppressant is selected from the group consisting of cyclosporin and rapamycin.
- Item 11:: The method as described in item 1 wherein said transplanted tissue is pancreatic tissue.
- Item 12:: The method as described in item 11 wherein said transplanted tissue comprises islet tissue.

### Example

The following example demonstrates that treating islet allograft recipient mice with anti-IL-12 mAb is highly effective in producing Th1 to Th2 immune deviation in several model systems (i.e., fully MHC, partially MHC, or multiple minor Ag barriers). Nevertheless, anti-IL-12 failed to prolong the engraftment of fully MHC-mismatched islet allografts. However, anti-IL-12-treated recipients carrying MHC-matched but multiple minor Ag-mismatched allografts experienced prolonged engraftment; allograft tolerance was frequently achieved in the DBA/2J (H-2d) to BALB/c (H-2d) strain combination. In another model, in which the host response was dominated by CD4+ T cells responding to donor allopeptides presented upon host APCs in the context of self MHC class II molecules, anti-IL-12 treatment proved to be extremely potent. Thus, Th1 to Th2 immune deviation produces prolonged engraftment as compared with recipients of MHC-mismatched allografts when rejection is dependent upon indirectly presented allogeneic peptides and a reduced mass of responding alloreactive T cells.

### Materials and Methods

### Reagents

A B cell hybridoma producing a depleting rat anti-mouse CD8 (2.43, IgG2a) was purchased from American Type Culture Collection (ATCC, Manassas, VA). Ascites was prepared in nude mice and used in the study. A neutralizing rat anti-mouse IL-12 mAb (C17.15, IgG2a) that recognizes an epitope on the p40 subunit ofIL-12 was kindly provided by the Genetic Institute (Cambridge, MA). Rat IgG2a was used as an isotype control Ab.

### Animals

We obtained 8- to 10-wk-old male B10.BR (H-2k), CBA/ca (H-2k), DBA/2J (H-2d), B10.D2 (H-2d), and B6AF1 (H-2b/k.d) mice from the Jackson Laboratory (Bar Harbor, ME). Male BALB/c (H-2d) mice were purchased from Harlan Sprague-Dawley (Indianapolis, IN). MHC class II-deficient mice (C57BL/6Ab-/-) were obtained from Taconic (Germantown, NY). All animals were housed under standard conditions.

### Islet transplantation

Islet transplantation was performed as described previously (18) with some modifications. Donor pancreata were perfused in situ with 3.5 ml of type IV collagenase in HBSS (2 mg/ml; Worthington Biochemical, Freehold, NJ) through the common bile duct. The pancreata were harvested after perfusion and incubated at 37°C for 40 min. Crude islets were released from the pancreata by gentle vortex and further purified on discontinuous Percoll gradients. Briefly, the crude islet preparation was resuspended in 4 ml of 25% Percoll, and layered on top with 2 ml of 23% Percoll followed by 2 ml of 21% and 2 ml of 11 % Percoll gradients. After centrifugation at 1800 rpm for 10 min, the islets were harvested from the 21/11% gradient interface and washed twice in HBSS. A total of 300 to 400 islets were transplanted under the renal capsule of each recipient that had been rendered diabetic by a single i.p. injection of streptozotocin (225 mg/kg; Sigma, St. Louis, MO). Allograft function was monitored by serial blood glucose measurements using an Accu-Chek III blood glucose monitor (Boehringer Mannheim, Indianapolis, IN). Primary graft function was defined as a blood glucose level of <200 mg/dl on day 3 posttransplantation, and graft rejection was defined as a rise in blood glucose level of >300 mg/dl following a period of primary graft function.

### Histopathology

The left kidney bearing the islet transplants was removed from recipient mice at various times after transplantation, fixed in 10% formalin, and embedded in paraffin. Serial tissue sections (5 µm) were cut and mounted on Superfrost Plus glass slides (Fisher Scientific, Pittsburgh, PA), fixed in methanol, and stained in hematoxylin and eosin for the identification of the composition of the cellular infiltrates.

### Thymectomy and depletion of CD8+ T cells

Thymectomy was performed on adult recipient BALB/c mice using a pipette suction technique. Briefly, BALB/c mice underwent upper median stemotomy under general anesthesia, and the thymus was removed by gentle aspiration. The skin was then closed with interrupted sutures. At 7 to 10 days after thymectomy, mice were treated with a depleting anti-CD8 mAb (2.43; ATCC) that was administered i.p. (0.2 mg) on days -6, -3, and -1 before transplantation as described previously (26).

### Flow cytometry

Two-color cell staining was performed. Briefly, peripheral blood leukocytes (1 × 106) were stained with a biotinylated rat anti-mouse CD4 mAb (GK1.5) on ice for 45 min, followed by staining with FITC-conjugated streptavidin (PharMingen, San Diego, CA). Cells were washed twice in PBS-0.5% BSA and further stained with a phycoerythrin-conjugated rat anti-mouse CD8 mAb (53-6.7; PharMingen). Isotype-matched, fluorescent-conjugated anti-2,4,6-trinitrophenyl mAbs (PharMingen) were used as controls. Cells were then washed in PBS and analyzed using a FACScan flow cytometer (Becton Dickinson, Mountain View, CA).

### RNA extraction

Tissue samples were homogenized, and total cellular RNA was extracted using a Qiagen RNA isolation kit (Chatsworth, CA) according to the manufacturer's instructions. RNA was reverse transcribed into cDNA in a 40-µl reaction mixture containing first strand buffer (10 mM Tris-HCl, 15 mM KCI, and 0.75 mM MgC12), 10 mM of deoxynucleoside triphosphate mix, 100 mM of DTT, 5 U of RNase inhibitor (31 U/µl RNasin; Pharmacia, Uppsala, Sweden), 1 µg/µl of BSA, 500 µg/ml of random primers, and 200 U of Moloney murine leukemia virus reverse transcriptase (Life Technologies, Grand Island, NY). The reaction mixture was incubated at 37°C for 1 h, terminated by heat inactivation at 65°C for 10 min, and stored at -20°C.

### Quantitative RT-PCR (QRT-PCR)

QRT-PCR was performed as reported previously (27) with some modifications. Briefly, 1 µl of reverse-transcribed cDNA was coamplified with a known concentration of gene-specific competitive template in a 50-µl reaction volume containing 10 mM of deoxynucleoside triphosphate, 100 ng of sense and antisense primers, and 0.25 U of Taq polymerase (Promega, Madison, WI). The specific primers for murine IL-2, IL-4, IL-10, IFN-, and glyceraldehyde-3-phosphate dehydrogenase (GAPDH) cDNA were used as reported previously (18). The gene-specific DNA competitors (bearing a 60-80-bp deletion from wild-type (wt) cDNA) were generated from Con A-stimulated splenic leukocytes using specially designed double sense primers. All competitors were cloned in a TA cloning vector (Invitrogen, San Diego, CA), transfected into DH5 cells (Life Technologies), purified, and quantitated with a UV spectrophotometer (Beckman, Columbia, MD).

The PCR amplification schema consisted of the following cycle components: denaturing at 94°C for 1 min, annealing at 55°C for 45 s, and extension at 72°C for 1 min for each cycle in a thermocycler (Perkin-Elmer Cetus, Norwalk, CT) for a total of 40 cycles. A positive and a negative control were included for each PCR amplification. Negative controls were performed by omitting the initial cDNA in the PCR reaction mixture. As a positive control, cDNA from Con A-stimulated splenic mononuclear leukocytes was used. In addition, amplification of the constitutively expressed GAPDH gene served to confirm the successful isolation and reverse transcription of total cellular RNA. PCR products were analyzed in ethidium bromide-stained 2% agarose gel and photographed with Polaroid positive/negative films (Polaroid 55, Cambridge, MA) under UV light. The PCR results on the negative film were scanned into a computer using a desktop scanner, and the density of the wt cDNA and the gene-specific competitor bands was analyzed using ImmunoQuan computer software (version 1.1, Hercules, CA). The magnitude of target gene expression is calculated and expressed as picograms of target gene cDNA per picogram of GAPDH cDNA in each sample.

### Serum IgG isotype assay

A sandwich ELISA was used to assess serum IgG1 or IgG2a titers. Briefly, 96-well plates were coated with rabbit anti-mouse IgG at 5 µg/ml (50 µl/well) at 4°C overnight and subsequently blocked with 0.5% BSA in PBS at room temperature for 1 h. Plates were washed three times with PBS-Tween 20 and then incubated with serial diluted serum (50 µl/well) at 4°C overnight. After washing in PBS-Tween 20, isotype-specific rat anti-mouse Ab was added (5 µg/ml in blocking buffer, 50 µl/well; Zymed Laboratories, Palo Alto, CA), and the plates were incubated at 4°C overnight followed by washing three times in PBS-Tween 20. An alkaline phosphatase-conjugated goat anti-rat IgG (1:1000 dilutions) was added and further incubated at 4°C for 2 h. The reaction was developed by the addition of alkaline phosphatase substrate and was quantitated in an ELISA reader at 405 nm.

### Statistics

Graft survival was analyzed using the log-rank test; p < 0.05 was considered significant.

### Results

### Anti-IL-12 mAb induced a Th1 to Th2 immune deviation in recipient mice

To determine whether treating recipient mice with anti-IL-12 mAb at the time of islet transplantation can induce Th1 to Th2 immune deviation, crude islet allografts were transplanted into fully MHC-mismatched or MHC-matched but multiple minor histocompatibility Ag-mismatched recipient mice. The recipient mice were administered 1 mg of anti-IL-12 mAb i.p. on days 0, 1, and 7 posttransplant, and the intragraft expression of Th1 (IL-2 and IFN-) and Th2 (IL-4 and IL-10) genes was analyzed by QRT-PCR. The timepoints chosen for this analysis correlated with the time of maximal leukocytic infiltration in the grafts of untreated recipients as assessed by histology (i.e., day 8 posttransplant on MHC-mismatched grafts and day 16 on minor Ag-mismatched grafts). As shown in Figure 1, a vigorous expression of IL-2 and IFN- was detected in rejecting MHC-mismatched B10.BR (H-2k) islet allografts by BALB/c (H-2d) recipients, whereas IL-4 transcripts were barely detectable. In anti-IL-12-treated recipients, however, the intragraft expression of IL-4 and IL-10 was markedly increased, and the expression of IL-2 and IFN- was proportionally down-regulated. Thus, Th1 to Th2 deviation was manifested following anti-IL-12 treatment. Similarly, the rejection of minor Ag-mismatched DBA/2J (H-2d) islet allografts by BALB/c (H-2d) recipients was also associated with a robust intragraft expression of IL-2 and IFN-, while anti-IL-12 treatment produced an increased expression of IL-4 and IL-10 and a proportional decrease of IL-2 and IFN- expression (Fig. 2).

An analysis of serum IgG isotype also demonstrated an increased titer of IgG1, which is often associated with a Th2 immune response, at 14 days after anti-IL-12 treatment in recipient mice (data not shown). Thus, anti-IL-12 is highly effective in inducing a Th1 to Th2 immune deviation as well as a shift of the IgG1 isotype of Ab production in recipient mice receiving either MHC-mismatched or MHC-matched but minor Ag-mismatched allografts.

### Anti-IL-12 treatment resulted in prolonged engraftment of MHC-matched and multiple minor Ag-mismatched but not MHC-mismatched islet allografts

Table I summarizes the graft survival times of islet allografts that were transplanted across major or minor histocompatibility barriers. Fully MHC-mismatched B10.BR (H-2k) islet allografts were rejected by BALB/c (H-2d) recipients with a mean survival time (MST) of 15 days (15 ± 2, n = 5). Treatment with anti-IL-12 failed to prolong allograft survival (MST = 13 ± 3, n = 5) despite immune deviation to a Th2-dominated allograft response (Fig. 1). Partially MHC-mismatched DBA/2J (H-2d) islet allografts were rejected by isotype control Ab-treated B6AF1 (H-2b/k.d) recipients with an MST of 17 days (17 ± 5, n = 6). Anti-IL-12 mAb treatment, which induced a Th1 to Th2 deviation in recipient mice as assessed by intragraft gene expression for IL-4 and IFN- (data not shown), produced a modest prolongation of DBA/2J islet allografts (25 ± 6, n = 6) (p < 0.05), but all of the islet allografts were eventually rejected (Table I).

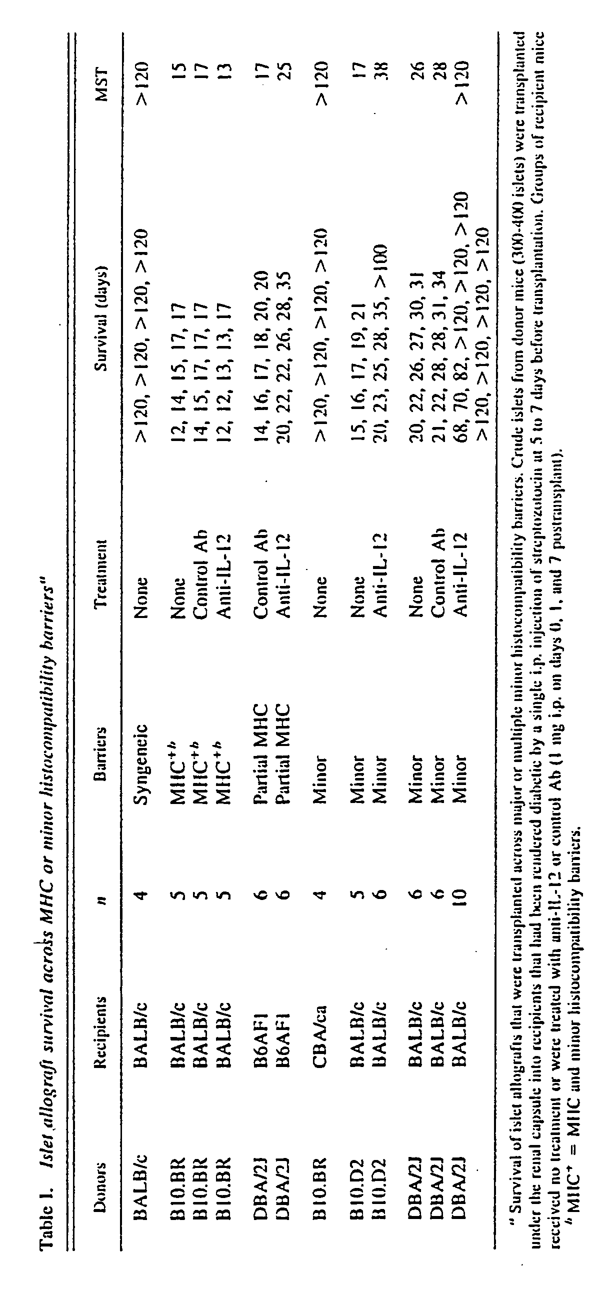

The rejection of MHC-matched but multiple minor Ag-mismatched islet allografts exhibited remarkable variation between different strain combinations. B10.BR (H-2k) islet allografts were permanently accepted by CBA/ca (H-2k) recipients without any therapeutic intervention (>120 days, n = 4), although skin grafts are vigorously rejected in this strain combination (24). In contrast, DBA/2J (H-2d) islet allografts were rejected by BALB/c (H-2d) recipients with an MST of 26 days (26 ± 4, n = 6). Interestingly, BALB/c (H-2d) recipients rejected multiple minor Ag-mismatched B10.D2 (H-2d) islets with considerable vigor (MST = 17 ± 3, n = 5) (Table I).

In contrast to the effect of anti-IL-12 treatment on the survival of MHC-mismatched allografts, anti-IL-12 mAb treatment of BALB/c recipients enabled a prolonged engraftment of multiple minor Ag-mismatched B 10.D2 islet allografts (MST > 38 days, n = 6) and the indefinite survival of DBA/2J islet allografts. In fact, 7 of 10 BALB/c recipient mice with DBA/2J islets survived for >120 days, while the other 3 mice were rejected on days 68, 70, and 82 posttransplant, respectively. Control mAb-treated BALB/c recipients rejected DBA/2J islet allografts at 28 days (28 ± 6, n = 6). Histologic examination of the islet allografts that were harvested at 120 days posttransplantation showed sporadic focal lymphocytic infiltration surrounding but not invading the islet allografts; this finding is in striking contrast with the control Ab-treated mice, in which lymphocytic invasion and islet destruction were extensive (data not shown).

Nephrectomy was performed (DBA/2J islet allografts were placed under the renal capsule) on five BALB/c recipient mice at 120 days posttransplantation to determine whether tolerance is evident in anti-IL-12-treated recipient mice bearing long-term surviving MHC-matched but minor Ag-mismatched allografts. As shown in Figure 3, removal of the left kidney bearing the DBA/2J islet allografts resulted in a sharp rise in blood glucose levels; these levels were >350 mg/dl after 2 days, demonstrating that the euglyceinia of these mice was maintained by the islet allografts. The nephrectomized BALB/c mice accepted a second DBA/2J islet allograft without any immunosuppression, indicating a state of tolerance.

### Anti-IL-12 treatment induced prolonged engraftment of islet allografts from MHC class II-deficient C57BL/6 donors in thymectomized and CD8+ cell-depleted BALB/c recipients

Clearly, a Th1 to Th2 immune deviation induces a prolonged engraftment of islet allografts that have been transplanted across multiple minor but not major histocompatibility barriers. As the rejection of MHC-matched but multiple minor Ag-mismatched allografts is mediated primarily by the indirect presentation of alloantigens via a smaller mass of responding T cell clones than MHC-mismatched allografts (28, 29), we reasoned that the recognition of donor MHC alloantigens in the context of "self" MHC may also enable tolerance to be established via immune deviation in this model. In this circumstance of indirect Ag presentation, the number of T cell clones proliferating in the allograft response is much smaller than that responding to directly presented, intact, foreign MHC molecules. As a stringent test of this hypothesis, islet allografts from MHC class II knockout (KO) C57BL/6 (H-2b) mice were transplanted into BALB/c recipient mice (H-2d). The recipient mice were thymectomized, and CD8+ cells were depleted. Thus, the activation of recipient CD4+ T cells in this model is dominated by an indirect recognition of the donor alloantigens presented by recipient APCs. As shown in Figure 4A, control Ab-treated mice rejected the islet allografts at 28 days (MST = 28 ± 12, n = 5), confirming that indirect Ag presentation does play an important role in islet allograft rejection (30). FACS analysis of peripheral blood at the time of graft loss demonstrated a complete depletion of CD8+ T cells, while the composition of CD4+ T cells in the recipient mice was not altered (Fig. 5). The administration of anti-IL-12, which is very effective in inducing a Th1 to Th2 immune deviation in this model (Fig. 4B), induced a prolonged engraftment of MHC class II-deficient islet allografts. Three of five islet allografts survived >100 days; the remaining two were rejected on days 52 and 66 posttransplant, respectively (Fig. 4A). In contrast, islets from wt C57BL/6 donors were vigorously rejected by unmodified BALB/c recipient mice that had been treated with anti-IL-12 (11 ± 2, n = 4) or by thymectomized BALB/c recipients without CD8 depletion that had been treated with anti-IL-12 (12 ± 4, n = 6).

### Discussion

Since the discovery of the cross-regulating Th1 and Th2 phenotypes and the elucidation of the immunoregulatory effects of Th2 in autoimmunity and infectious diseases, there has been great anticipation that a Th1 to Th2 immune deviation may be critical in the acquisition of transplantation tolerance. Although tolerizing therapy in some animal models often skewed the immune activation toward a Th2-dominated response (13, 14), extensive in-depth studies have repeatedly demonstrated that a Th1 to Th2 immune deviation does not uniformly permit the acquisition of transplant tolerance (31, 32). In the present study, we have sought to determine the basis for the frequent failure of Th1 to Th2 immune deviation and of Th2 cytokines to blunt the severity of allograft rejection despite the fact that such measures have proven highly effective in the treatment of many T cell-dependent autoimmune states (6, 8, 33). We have reasoned that alloimmunity and autoimmunity differ in terms of the nature of Ag presentation and the clonal size of responding T cells (29). The host response to MHC-mismatched allografts encompasses an unusually large population of T cell clones. Moreover, the direct recognition of foreign MHC during the allograft response has no counterpart in autoimmune states, in which autoantigens are processed by self APCs and presented in the context of self MHC. We have sought to determine whether these rather unique facets of the allograft response are responsible for the failure to prevent allograft rejection through Th1 to Th2 immune deviation.

Clearly, treatment with anti-IL-12 mAb at the time of transplantation in this islet allograft model is highly effective in inducing a Th2-type response, regardless of histocompatibility barriers (i.e., fully MHC, partially MHC, or multiple minor Ag barriers) (Figs. 1 and 2). While the impact of anti-IL-12 upon the pattern of cytokine expression was consistent, stunning differences were noted in the impact of such therapy upon the duration of engraftment in MHC-mismatched vs MHC-matched but multiple minor Ag-mismatched conditions. In accordance with the observations of Piccotti et al. using a cardiac allograft model (22), anti-IL-12 treatment was totally ineffective in prolonging the engraftment of MHC-mismatched islet allografts (Table I). With regard to the mode of Ag presentation and the responding T cell clonal size, the allograft response to MHC-matched but minor Ag-mismatched allografts may more closely resemble the response to autoantigens than the response to MHC-mismatched grafts. Unlike the responses to MHC-mismatched allografts, foreign peptides are presented on self genotype MHC molecules. Thus, Ag presentation, as in the case of autoimmune reactions, is indirect. Moreover, a larger T cell clonal mass is stimulated by MHC-mismatched allografts than by minor Ag-mismatched allografts. Consequently, we were fascinated to learn that most anti-IL-12-treated recipients carrying MHC-matched but multiple minor Ag-mismatched allografts experienced a prolonged engraftment; allograft tolerance was frequently achieved in the DBA/2J to BALB/c strain combination (Table I and Fig. 3). Consistent with the failure of anti-IL-12 to produce benefits among MHC-mismatched allograft recipients and produce the acquisition of tolerance in similarly treated recipients of MHC-matched but minor Ag-mismatched allografts, anti-IL-12-treated recipients of partially MHC-mismatched allografts experienced a modest albeit significant increase in the duration of engraftment (Table I).

To further test the hypothesis that the mode of Ag presentation (direct vs indirect) and/or the size of the responding T cell mass underlie the failure of anti-IL-12 or other modes of producing Th1 to Th2 immune deviation to benefit the recipients of MHC-mismatched allografts, we studied the impact of anti-IL-12 treatment in an unusual MHC-mismatched allograft model. MHC class II KO C57BL/6 (H-2b) mice were used as donors, and MHC-mismatched, thymectomized BALB/c (H-2d) mice that had been treated with a depleting anti-CD8 protocol were used as recipients. As a consequence, the host response should be dominated by CD4 cells responding to the donor allopeptides that are presented upon host APCs in the context of self MHC class II molecules. The impact of anti-IL-12 treatment in the model closely resembled the potent effects that were noted in MHC-matched but multiple minor Ag-mismatched allograft recipients (Fig. 4A). In a control experiment, MHC class II-positive wt C57BL/6 donor allografts were rapidly rejected by unmodified (nonthymectomized, non-CD8-depleted) BALB/c hosts that had been treated with anti-IL-12 mAb.

In consideration of our results, the clearest example of IL-4-dependent allograft tolerance (infectious tolerance) was obtained in a model using MHC-matched multiple minor Ag-mismatched skin allografts (24). Hence, we conclude that immune deviation from a Th1 to a Th2 response does not universally blunt cytopathic T cell-dependent immune reactions. Rather, unique features of the T cell-dependent response to MHC-mismatched allografts render the alloimmune response resistant to the frequently seen tolerizing effects of Th1 to Th2 immune deviation (e.g. autoimmunity and neonatal tolerance) (23, 34). The implication of these results upon clinical strategies seems self-evident, although the precise mechanism for the failure of Th1 to Th2 immune deviation to curb allograft rejection in MHC-mismatched allograft recipients will require further investigation.

### References

1. Mosmann, T. R., and R. L. Coffman. 1989. Th1 and Th2 cells: different patterns of lymphokine secretion lead to different functional properties. Annu. Rev. Immunol. 7:145.
2. Trinchieri, G. 1995. IL-12, a proinflammatory cytokine with immunoregulatory functions that bridge innate resistance and antigen-specific adaptive immunity. Annu. Rev. Immunol. 13:251.
3. Magram, J., S. E. Connaughton, R R. Warrier, D. M. Carvajal, C. Wu, J. Ferrante, C. Stewart, U. Sarmiento, D. A. Faherty, and M. K. Gately. 1996. IL-12-deficient mice are defective in IFN- production and type 1 cytokine responses. Immunity 4:471.
4. Wu, C., J. Ferrante, M. K. Gately, and J. Magram. 1997. Characterization of IL-12 receptor 1-chain-deficient mice: IL-12 receptor 1 is an essential component of the functional mouse IL-12 receptor. J. Immunol. 159:1658.
5. Kaplan, M. H., Y. L. Sun, T. Hoey, and M. J. Grusby. 1996. Impaired IL-12 responses and enhanced development of Th2 cells in Stat4-deficient mice. Nature 382:174.
6. Kuchroo, V. K., M. P. Das, J. A. Brown, A. M. Ranger, S. S. Zamvil, R. A. Sobel, H. L. Weiner, N. Nabavi, and L. H. Glimcher. 1995. B7-1 and B7-2 costimulatory molecules activate differentially the Th1/Th2 development pathways: application to autoimmune disease therapy. Cell 80:707.
7. Mueller, R., T. Krahl, and N. Sarvetnick. 1996. Pancreatic expression of interleukin-4 abrogates insulitis and autoimmune diabetes in NOD mice. J. Exp. Med. 184:1093.
8. Zheng, X. X., A. W. Steele, W. W. Hancock, A. C. Stevens, P. W. Nickerson, P. Roy-Chaudhury, Y. Tian, and T. B. Strom. 1997. A noncytolytic IL-10/Fc fusion protein prevents diabetes, blocks autoimmunity, and promotes suppressor phenomena in NOD mice. J. Immunol. 158:4507.
9. Williamson, E., P. Garside, J. A. Bradley, I. A. R. More, and A. M. Mowat. 1997. Neutralizing IL-12 during induction of murine acute graft-versus-host disease polarizes the cytokine profile toward a Th2-type alloimmune response and confers long-term protection from disease. J. Immunol. 159:1208.
10. Leonard, J. P., K. E. Waldburger, and S. J. Goldman. 1995. Prevention of experimental autoimmune encephalomyelitis by antibodies against interleukin-12. J. Exp. Med. 181:381.
11. Neurath, M. F., I. Fuss, B. L. Kelsall, E. Stuber, and W. Strober. 1995. Antibodies to interleukin-12 abrogate established experimental colitis in mice. J. Exp. Med. 182:1281.
12. Gavett, S. H., D. J. O'Hearn, X. Li, S. K. Huang, F. D. Finkelman, and M. Wills-Karp. 1995. Interleukin 12 inhibits antigen-induced airway hyperresponsiveness, inflammation, and Th2 cytokine expression in mice. J. Exp. Med. 182:1527.
13. Takeuchi, T., R. P. Lowry, and B. Konieczny. 1992. Heart allografts in murine systems: the differential activation of Th2-like effector cells in peripheral tolerance. Transplantation 53:1281.
14. Chen, N., Q. Gao, and E. H. Field. 1996. Prevention of Th1 responses is critical for tolerance. Transplantation 61:1076.
15. Onodera, K., W. W. Hancock, E. Graser, M. Lehmann, M. H. Sayegh, T. B. Strom, H. D. Volk, and J. W. Kupiec-Weglinski. 1997. Type 2 helper T cell-type cytokines and the development of "infectious" tolerance in rat cardiac allograft recipients. J. Immunol. 158:1572.
16. Qin, L., K. D. Chavin, Y. Ding, H. Tahara, J. P. Favaro, J. E. Woodward, T. Suzuki, P. D. Robbins, M. T. Lotze, and J. S. Bromberg. 1996. Retrovirus-mediated transfer of viral IL-10 gene prolongs murine cardiac allograft survival. J. Immunol. 156:2316.
17. Sayegh, M. H., E. Akalin, W. W. Hancock, M. E. Russell, and C. B. Carpenter. 1995. CD28-B7 blockade after alloantigenic challenge in vivo inhibits Th1 cytokines but spares Th2. J. Exp. Med. 181:1869.
18. Steiger, J., P. W. Nickerson, W. Steurer, M. Moscovitch-Lopatin, and T. B. Strom. 1995. IL-2 knockout recipient mice reject islet cell allografts. J. Immunol. 155:489.
19. Nickerson, P. W., X. X. Zheng, J. Steiger, A. W. Steele, W. Steurer, P. Roy-Chaudhury, W. Muller, and T. B. Strom. 1996. Prolonged islet allograft acceptance in the absence of interleukin-4 expression. Transpl. Immunol. 4:81.
20. Lakkis, F. G., B. T. Konieczny, S. Saleem, F. K. Baddoura, P. S. Linsley, D. Z. Alexander, R. P. Lowry, T. C. Pearson, and C. P. Larsen. 1997. Blocking the CD28-B7 T cell costimulation pathway induces long-term cardiac allograft acceptance in the absence of IL-4. J. Immunol. 158:2443.
21. Zheng, X. X., A. W. Steele, P. W. Nickerson, W. Steurer, J. Steiger, and T. B. Strom. 1995. Administration of noncytolytic IL-10/Fc in murine models of lipopolysaccharide-induced septic shock and allogeneic islet transplantation. J. Immunol. 154:5590.
22. Piccotti, J. R., S. Y. Chan, R. E. Goodman, J. Magram, E. J. Eichwald, and D. K. Bishop. 1996. IL-12 antagonism induces Th2 responses, yet exacerbates cardiac allograft rejection. J. Immunol. 157:1951.
23. Gao, Q., N. Chen, T. M. Rouse, and E. H. Field. 1996. The role of interleukin-4 in the induction phase of allogeneic neonatal tolerance. Transplantation 62:1847.
24. Davies, J. D., G. Martin, J. Phillips, S. E. Marshall, S. P. Cobbold, and H. Waldmann. 1996. T cell regulation in adult transplantation tolerance. J. Immunol. 157:529.
25. Donckier, V., M. Wissing, C. Bruyns, D. Abramwicz, M. Lybin, M. L. Vanderhaeghen, and M. Goldman. 1995. Critical role of interleukin-4 in the induction of neonatal transplantation tolerance. Transplantation 59:1571.
26. Lee, R. S., M. J. Grusby, L. H. Glimcher, H. J. Winn, and H. Auchincloss, Jr. 1994. Indirect recognition by helper cells can induce donor-specific cytotoxic T lymphocytes in vivo. J. Exp. Med. 179:865.
27. Strehlau, J., M. Pavlakis, M. Lipman, M. Shapiro, L. Vasconcellos, W. Harmon, and T. B. Strom. 1997. Quantitative detection of immune activation transcripts as a diagnostic tool in kidney transplantation. Proc. Natl. Acad. Sci. USA 94:695.
28. Warrens, A. N., G. Lombardi, and R. I. Lechler. 1994. Presentation and recognition of major and minor histocompatibility antigens. Transpl. Immunol. 2:103.
29. Shoskes, D. A., and K. J. Wood. 1994. Indirect presentation of MHC antigens in transplantation. Immunol. Today 15:32.
30. Stock, P. G., N. L. Ascher, S. Chen, J. Field, F. H. Bach, and D. E. Sutherland. 1991. Evidence for direct and indirect pathways in the generation of the alloimmune response against pancreatic islets. Transplantation 52:704.
31. Strom, T. B., P. Roy-Chaudhury, R. Manfro, X. X. Zheng, P. W. Nickerson, K. Wood, and A. Bushell. 1996. The Th1/Th2 paradigm and the allograft response. Curr. Opin. Immunol. 8:688.
32. Piccotti, J. R., S. Y. Chan, A. M. VanBuskirk, E. J. Eichwald, and D. K. Bishop. 1997. Are Th2 helper T lymphocytes beneficial, deleterious, or irrelevant in promoting allograft survival? Transplantation 63:619.
33. Mueller, R., J. D. Davis, T. Krahl, and N. Sarvetnick. 1997. IL-4 expression by grafts from transgenic mice fails to prevent allograft rejection. J. Immunol. 159:1599. [Medline] 34.Seder, R. A., B. L. Kelsall, and D. Jankovic. 1996. Differential roles for IL-12 in the maintenance of immune responses in infectious versus autoimmune disease. J. Immunol. 157:2745.

## Claims

1. Use of a therapeutically effective amount of an anti-IL 12 antibody for the preparation of a pharmaceutical composition for reducing or ameliorating chronic rejection of a transplanted tissue in a mammalian subject, wherein said tissue has a mismatch of minor alloantigens with said subject.

2. The use of claim 1, wherein said antibody recognizes an epitope on a p40 subunit.

3. The use of any one of claims 1 to 2, wherein said subject is human.

4. The use of any one of claims 1 to 3, wherein said antibody is administered in combination with at least one other therapeutic agent.

5. The use of claim 4, wherein said other therapeutic agent reduces or eliminates a secondary co-stimulatory immune signal.

6. The use of claim 5, wherein said therapeutic agent is a soluble form of CTLA4, an antibody directed to B7.1, an antibody directed to B7.2 or an antibody directed to CD28.

7. The use of any one of claims 1 to 6, wherein said antibody is a polyclonal antibody, a monoclonal antibody, a chimeric antibody, a single-chain antibody, a CDR-grafted antibody, a humanized antibody or fragments thereof which bind IL-12.

8. The use of any one of claims 1 to 7, wherein said antibody is a human antibody.

9. The use of claim 4 or 5, wherein said other therapeutic agent is an immunosuppressant.

10. The use of claim 9, wherein said immunosuppressant is cyclosporin or rapamycin.
